# EUROPEAN PATENT APPLICATION

(11) **EP 4 491 181 A1**
(43) Date of publication of application: **15.01.2025**
(21) Application number: 22930375.5
(22) Date of filing: 17.03.2022
(51) Int. Cl.: A61K 31/47, A61K 31/4709, A61K 31/496, A61K 31/5377, A61P 35/00

(54) **NOVEL USE OF QUINOLINE COMPOUND**

(30) Priority: 08.03.2022 CN 202210227577
(71) Applicant: Tarapeutics Science Inc., Bengbu, Anhui 233000 (CN)
(72) Inventor: ZHANG, Wentao, Bengbu, Anhui 233000 (CN); JIANG, Yunyun, Bengbu, Anhui 233000 (CN)
(74) Representative: Abel & Imray LLP
(86) International application number: PCT/CN2022/081439
(87) International publication number: WO 2023/168740

(57) **Abstract**

A compound of formula (I) or a pharmaceutically acceptable salt, solvate, ester, acid, metabolite or prodrug thereof, which is used for treating in a tumor microenvironment cancers or tumors where M2-phenotype macrophages predominate, Y, A, n, R₁, R₂ and R₃ being as defined in the description. By converting M2-phenotype TAMs into M1-phenotype TAMs in a tumor immune microenvironment, the tumor microenvironment is improved, the phagocytic effect of macrophages on tumor cells is promoted, and the growth of the tumor is further inhibited.

## Description

### Technical Field

The invention relates to the field of biological medicine, and specifically relates to the use of a class of quinoline compounds in anticancer drugs.

### Background of the Invention

Cancer immunotherapy is an emerging and most promising treatment method after surgery, chemoradiotherapy and targeted therapy, and it is also the focus and hotspot in the current tumor research field. However, a large proportion of patients in clinical cases are still insensitive to immunotherapy, which has been proven to be inseparable from the immune tolerance mediated by the tumor micro environment (TME). Targeting TME to relieve immunosuppression is conducive to restoring and rebuilding the body's normal anti-tumor immune defense ability and enhancing the efficacy of immunotherapy.

Tumor associated macrophages (TAMs) are an important part of TME, which play an important role in the occurrence, development, infiltration and metastasis of tumors. Macrophages can be divided into M1 (classically activated macrophages) and M2 (alternatively activated macrophages) according to their phenotype and secreted cytokines.

M1 macrophages mainly secrete proinflammatory cytokines and chemokines, thereby enhancing antigen presentation and promoting proliferation and activation of T cells, participating in inflammatory response and thus improving the immune killing ability against tumor cells. In contrast, M2 macrophages have weak antigen-presenting capacity, can down-regulate immune response and inhibit the cytotoxicity of CD8+T in the TME, which can promote the occurrence and development of tumors. M1 macrophages can be identified by indicators such as membrane proteins CD80/86, and cytokines IL-1β, IL-6 secreted by them; while M2 macrophages can be identified by indicators such as the expression amount of Arg-1 and CD206/163.

A large number of studies have found that TAMs in tumor tissues are mainly M2, which is an important factor in the formation of the immunosuppressive TME. Moreover, some studies have shown that specific stimuli can directionally induce the transformation of M2 TAMs into M1 TAMs, thereby inhibiting tumor growth and enhancing cancer immunotherapy efficacy. Therefore, with TAMs as the target, exploiting regulatory factors that can promote the polarization of TAMs to M1 and promote the transformation of TAMs from M2 to M1 will provide new ideas for future tumor immunotherapy. This class of drugs can significantly inhibit tumor growth in clinical practice, and even some solid tumors may completely disappear after treatment.

### Summary of the Invention

An object of the invention is to provide a class of compounds that induce the transformation of M2 TAMs into M1 TAMs, thereby inhibiting tumor growth and improving efficacy of cancer immunotherapy, and thus can be used to treat cancers or tumors in which M2 macrophages are dominant in the tumor microenvironment. In addition, an object of the invention is further to provide a class of compounds that can inhibit the activity of CSF1R kinase and related signaling pathways, thereby being able to activate autoimmunity for treatment of cancer by inhibiting the phosphorylation of CSF1R.

Therefore, the invention provides the use of a compound of Formula (I), or a pharmaceutically acceptable salt, solvate, ester, acid, metabolite or prodrug thereof, in preparation of a medicament for treating cancers or tumors in which M2 macrophages are dominant in the tumor microenvironment: 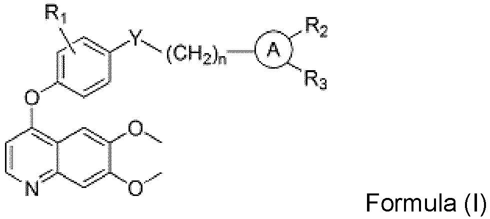
wherein, Y is selected from 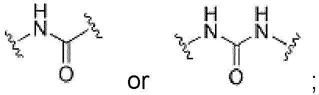
A is selected from aryl or 6-membered heterocyclyl;
n is an integer selected from 1-3;
R₁ is selected from hydrogen, halo, C₁₋₆alkyl, C₁₋₆haloalkyl, C₁₋₆alkoxy and cyano;
each of R₂ and R₃ is independently selected from hydrogen, halo, C₁₋₆alkyl, C₁₋₆haloalkyl, C₁₋₆alkoxy, or R₂ and R₃ together form a phenyl or 5-membered heterocyclyl.

In a preferred embodiment, n is preferably 1 when Y is 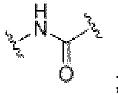 n is preferably 3 when Y is 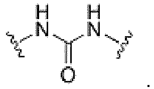 In another aspect, A is preferably selected from phenyl, N-morpholinyl, N-piperidyl or N-piperazinyl. Further preferably, R₁ is selected from hydrogen, fluoro, chloro, methyl, ethyl, propyl, or cyano; each of R₂ and R₃ is independently selected from hydrogen, fluoro, chloro, methyl, ethyl, propyl, trifluoromethyl, difluoromethyl, trifluoroethyl, methoxy, ethoxy, propoxy, or R₂ and R₃ together form a phenyl or dioxolane.

In a preferred embodiment, the invention provides the use of a compound of Formula (Ia), or a pharmaceutically acceptable salt, solvate, ester, acid, metabolite or prodrug thereof, in preparation of a medicament for treating cancers or tumors in which M2 macrophages are dominant in the tumor microenvironment: 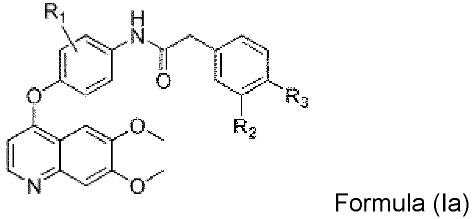 wherein, R₁, R₂ and R₃ are defined as above.

More preferably, wherein R₁ is selected from hydrogen, fluoro, chloro, and methyl; each of R₂ and R₃ is independently selected from hydrogen, fluoro, chloro, methyl, and trifluoromethyl, or R₂ and R₃ together form a phenyl.

In a further preferred embodiment, the invention provides the use of a compound of Formula (Ib), or a pharmaceutically acceptable salt, solvate, ester, acid, metabolite or prodrug thereof, in preparation of a medicament for treating cancers or tumors in which M2 macrophages are dominant in the tumor microenvironment: 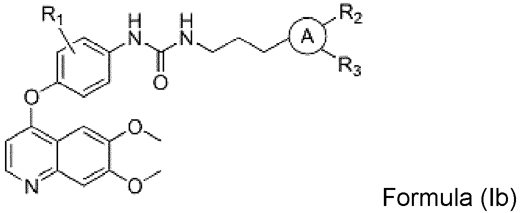 wherein, A, R₁, R₂ and R₃ are defined as above.

More preferably, wherein A is selected from phenyl or N-morpholinyl; R₁ is hydrogen; each of R₂ and R₃ is independently selected from hydrogen, fluoro, chloro, methyl, and trifluoromethyl.

The invention relates to any one of the compounds of Formula (I), Formula (Ia) and Formula (Ib), or a pharmaceutically acceptable salt, solvate, ester, acid, metabolite or prodrug thereof, which can transform macrophages in TME from M2 that promotes tumor growth to M1, thereby changing TME and achieving the purpose of treating cancer. In addition, the compounds of Formula (I), Formula (Ia) and Formula (Ib) of the invention, or pharmaceutically acceptable salts, solvates, esters, acids, metabolites or prodrugs thereof, can inhibit the activity of CSF1R kinase and related signaling pathways, thereby achieving the purpose of treating cancer.

The invention relates to any one of the compounds of Formula (I), Formula (Ia) and Formula (Ib), or a pharmaceutically acceptable salt, solvate, ester, acid, metabolite or prodrug thereof, which is used as medication for treating cancers or tumors in which M2 macrophages are dominant in the tumor microenvironment.

The cancer or tumor is selected from one or more of ovarian cancer, cervical cancer, membranous adenocarcinoma, prostate cancer, bladder cancer, lung cancer, thyroid cancer, breast cancer, pancreatic cancer, kidney cancer, gastric cancer, liver cancer, cervix cancer, endometrial cancer, colorectal cancer, nasopharyngeal cancer, esophageal cancer, bile duct cancer, bone metastatic cancer, papillary thyroid cancer, non-small cell lung cancer, small cell lung cancer, colon cancer, solid tumor, brain tumor, lymphoma, glioma, glioblastoma, melanoma, mesothelioma, glioblastoma, osteosarcoma, gastrointestinal stromal tumor, multiple myeloma, carcinosarcoma of the bile duct, and leukemia. Of these, the lymphoma comprises non-Hodgkin's lymphoma, B cell or T cell lymphoma; the leukemia comprises chronic myelocytic leukemia or acute myeloid leukemia.

The invention also relates to a method for treating cancers or tumors in which M2 macrophages are dominant in the tumor microenvironment, characterized in that, the method comprises administering to a patient any one of the compounds of Formula (I), Formula (Ia) and Formula (Ib) of the invention, or a pharmaceutically acceptable salt, solvate, ester, acid, metabolite or prodrug thereof.

### Description of the Figures

Figure 1 shows the effects of Compound 2, BLZ945 and PLX3397 on IL-10 secretion after polarization of RAW264.7 cells.
Figure 2 shows the effects of Compound 2, BLZ945 and PLX3397 on expression of the molecule CD86 after polarization of RAW264.7 cells.
Figure 3 shows the effects of Compound 2 and PLX3397 on mouse body weight after administration in a MC38 cell tumor transplantation mouse model.
Figure 4 shows the tumor inhibitory effect of Compound 2 and PLX3397 after administration in a MC38 cell tumor transplantation mouse model.
Figure 5 shows the effects of Compound 2 and PLX3397 on the proportion of M1 macrophages in the tumor microenvironment after administration in a MC38 cell tumor transplantation mouse model.
Figure 6 shows the effects of Compound 2 and PLX3397 on mouse body weight after administration in a 4T1 cell tumor transplantation mouse model.
Figure 7 shows the tumor inhibitory effect of Compound 2 and PLX3397 after administration in a 4T1 cell tumor transplantation mouse model.
Figure 8 shows the effects of Compound 2 and PLX3397 on the proportion of M1 macrophages in the tumor microenvironment after administration in a 4T1 cell tumor transplantation mouse model.
Figure 9 shows the effects of Compound 2 and PLX3397 on the proportion of M2 macrophages in the tumor microenvironment after administration in a 4T1 cell tumor transplantation mouse model.

### Detailed Description of the Invention

### Terminology

Unless defined otherwise, all technical and scientific terms used herein have the same meaning as generally understood by one skilled in the art of the subject matter claimed for protection.

Unless otherwise indicated, conventional methods such as mass spectroscopy, NMR, HPLC, protein chemistry, biochemistry, recombinant DNA techniques and pharmacology, which are within the skill of the art, are employed in the invention. Unless specific definitions are provided, the nomenclature related to, and the laboratory procedures and techniques of chemistry such as analytical chemistry, synthetic organic chemistry, and medicinal and pharmaceutical chemistry described herein are known to those skilled in the art. In general, the aforementioned techniques and procedures may be performed by conventional methods well known in the art and described in various general and more specific references which are referenced and discussed throughout the specification.

The term "alkyl" refers to an aliphatic hydrocarbon group, which may be a branched or linear alkyl. Depending on the structure, an alkyl may be a monovalent group or a divalent group (i.e., an alkylene group). In the invention, the alkyl group is preferably an alkyl having 1 to 8 carbon atoms, more preferably a "lower alkyl" having 1 to 6 carbon atoms, and even more preferably an alkyl having 1 to 4 carbon atoms. Typical alkyl groups include, but are not limited to, methyl, ethyl, propyl, butyl, pentyl, hexyl, and the like. It should be understood that the "alkyl" as mentioned herein encompasses all configurations and conformations that may exist of the alkyl, e.g., the "propyl" as mentioned herein intends to encompass n-propyl and isopropyl, the "butyl" encompasses n-butyl, isobutyl, and tert-butyl, the "pentyl" encompasses n-pentyl, isopentyl, neopentyl, tert-pentyl, and pent-3-yl.

The term "alkoxy" refers to a -O-alkyl group, where alkyl is as defined herein. Typical alkoxy groups include, but are not limited to, methoxy, ethoxy, propoxy, butoxy, pentyloxy, hexyloxy, and the like.

The term "cycloalkyl" refers to a monocyclic or polycyclic radical that contains only carbon and hydrogen. Cycloalkyl groups include groups having from 3 to 12 ring atoms. Depending on the structure, a cycloalkyl group can be a monovalent group or a divalent group (e.g., a cycloalkylene group). In the invention, the cycloalkyl group is preferably a cycloalkyl having 3 to 8 carbon atoms, and more preferably a "lower cycloalkyl" having 3 to 6 carbon atoms. Examples of cycloalkyl include, but are not limited to, cyclopropyl, cyclobutyl, cyclopentyl, cyclohexyl, cycloheptyl, cyclooctyl, cyclopentenyl, cyclohexenyl, cycloheptenyl, and adamantyl.

The term "aromatic group" refers to a planar ring having a delocalized π-electron system containing 4n+2 π electrons, where n is an integer. Aromatic rings can be formed by five, six, seven, eight, nine, or more than nine atoms. Aromatic groups can be optionally substituted. The term "aromatic group" includes both carbocyclic aryl (e.g., phenyl) and heterocyclic aryl (or "heteroaryl" or "heteroaromatic") groups (e.g., pyridine). The term includes monocyclic or fused-ring polycyclic (i.e., rings which share adjacent pairs of carbon atoms) groups.

As used herein, the term "aryl" refers to an aromatic ring wherein each of the atoms forming the ring is a carbon atom. Aryl rings can be formed by five, six, seven, eight, nine, or more than nine atoms. Aryl groups can be optionally substituted. Examples of aryl groups include, but are not limited to phenyl, naphthalenyl, phenanthrenyl, anthracenyl, fluorenyl, and indenyl. Depending on the structure, an aryl group can be a monovalent group or a divalent group (i.e., an arylene group).

The term "aryloxy" refers to a -O-aryl group, wherein aryl is as defined herein.

The term "heteroaryl" refers to an aryl group that includes one or more ring heteroatoms selected from nitrogen, oxygen and sulfur. An N-containing "heteroaryl" moiety refers to an aromatic group in which at least one of the skeletal atoms of the ring is a nitrogen atom. Depending on the structure, the heteroaryl group may be a monovalent group or a divalent group (i.e., a heteroarylene group). Examples of heteroaryl groups include, but are not limited to pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, indazolyl, indolizinyl, phthalazinyl, pyridazinyl, isoindolyl, pteridinyl, purinyl, oxadiazolyl, thiadiazolyl, furazanyl, benzofurazanyl, benzothienyl, benzothiazolyl, benzoxazolyl, quinazolinyl, naphthyridinyl, furopyridinyl, and the like.

As used herein, the term "heteroalkyl" refers to an alkyl group, as defined herein, in which one or more of the skeletal chain atoms is a heteroatom, e.g., oxygen, nitrogen, sulfur, silicon, phosphorus, or combinations thereof. The heteroatom(s) may be placed at any interior position of the heteroalkyl group or at the position at which the heteroalkyl group is attached to the remainder of the molecule.

As used herein, the term "heterocycloalkyl" or "heterocyclyl" refers to a non-aromatic ring wherein one or more atoms forming the ring is a heteroatom selected from the group consisting of nitrogen, oxygen and sulfur. Heterocycloalkyl rings can be formed by three, four, five, six, seven, eight, nine, or more than nine atoms. Heterocycloalkyl rings can be optionally substituted. Examples of heterocycloalkyls include, but are not limited to, lactams, lactones, cyclic imides, cyclic thioimides, cyclic carbamates, tetrahydrothiopyran, 4H-pyran, tetrahydropyran, piperidine, 1,3-dioxin, 1,3-dioxane, 1,4-dioxin, 1,4-dioxane, piperazine, 1,3-oxathiane, 1,4-oxathiin, 1,4-oxathiane, tetrahydro-1,4-thiazine, 2H-1,2-oxazine, maleimide, succinimide, barbituric acid, thiobarbituric acid, dioxopiperazine, hydantoin, dihydrouracil, morpholine, trioxane, hexahydro-1,3,5-triazine, tetrahydrothiophene, tetrahydrofuran, pyrroline, pyrrolidine, imidazolidine, pyrrolidone, pyrazoline, pyrazolidine, imidazoline, imidazolidine, 1,3-dioxole, 1,3-dioxolane, 1,3-dithiole, 1,3-dithiolane, isoxazoline, isoxazolidine, oxazoline, oxazolidine, oxazolidinone, thiazoline, thiazolidine, and 1,3-oxathiolane. Depending on the structure, a heterocycloalkyl group can be a monovalent group or a divalent group (i.e., a heterocycloalkylene group).

The term "halo" or "halogen" means fluoro, chloro, bromo and iodo.

The terms "haloalkyl", "haloalkoxy" and "haloheteroalkyl" include alkyl, alkoxy or heteroalkyl structures in which at least one hydrogen is replaced with a halogen atom. In certain embodiments in which two or more hydrogen atoms are replaced with halogen atoms, the halogen atoms are the same or different as one another.

The term "hydroxy" refers to an -OH group.

The term "cyano" refers to a -CN group.

The term "ester group" refers to a chemical moiety of the formula -COOR, wherein R is selected from alkyl, cycloalkyl, aryl, heteroaryl (connected via a ring carbon) and heterocyclyl (connected via a ring carbon).

The term "amino" refers to an -NH₂ group.

The term "aminoacyl" refers to a -CO-NH₂ group.

The term "amide" or "acylamino" refers to a -NR-CO-R' group, wherein each of R and R' is independently hydrogen or alkyl.

The term "optionally" means that the subsequently described event(s) may occur or may not occur, and encompasses both event(s) that occur, and event(s) that do not occur. The term "optionally substituted" or "substituted" means that the referenced group may be substituted with one or more additional group(s) individually and independently selected from the group consisting of alkyl, cycloalkyl, aryl, heteroaryl, heterocyclyl, hydroxy, alkoxy, cyano, halo, amide, nitro, haloalkyl, amino, mesyl, alkylcarbonyl, alkoxycarbonyl, heteroarylalkyl, heterocycloalkylalkyl, aminoacyl, amino-protecting group, and the like. Of these, the amino-protecting group is preferably selected from the group consisting of pivaloyl, tert-butoxycarbonyl, benzyloxycarbonyl, 9-fluorenylmethoxycarbonyl, benzyl, p-methoxybenzyl, allyloxycarbonyl, trifluoroacetyl, and the like.

The term "tyrosine protein kinase (TPK)" as used herein is a type of kinases that catalyze the transfer of the γ-phosphate from adenosine triphosphate (ATP) to tyrosine residue of proteins and that is capable of catalyzing the phosphorylation of tyrosine residue of various protein substrates, and thus has an important effect in cell growth, proliferation and differentiation.

The terms "inhibit", "inhibiting", or "inhibitor" of a kinase, as used herein, refer to inhibition of phosphotransferase activity.

A "metabolite" of a compound disclosed herein is a derivative of the compound that is formed when the compound is metabolized. The term "active metabolite" refers to a biologically active derivative of a compound that is formed when the compound is metabolized. The term "metabolized" as used herein, refers to the sum of the processes (including, but not limited to, hydrolysis reactions and reactions catalyzed by enzymes, such as, oxidation reactions) by which a particular substance is changed by an organism. Thus, enzymes may produce specific structural alterations to a compound. For example, cytochrome P450 catalyzes a variety of oxidative and reductive reactions while uridine diphosphate glucuronyl transferases catalyze the transfer of an activated glucuronic acid molecule to aromatic alcohol, aliphatic alcohol, carboxylic acid, amine and free sulfhydryl group. Further information on metabolism may be obtained from The Pharmacological Basis of Therapeutics, 9th Edition, McGraw-Hill (1996). Metabolites of the compounds disclosed herein can be identified either by administration of compounds to a host and analysis of tissue samples from the host, or by incubation of compounds with hepatic cells *in vitro* and analysis of the resulting compounds. Both methods are well known in the art. In some embodiments, metabolites of a compound are formed by oxidative processes and correspond to the corresponding hydroxy-containing compound. In some embodiments, a compound is metabolized to pharmacologically active metabolites. The term "modulate" as used herein, means to interact with a target either directly or indirectly so as to alter the activity of the target, including, by way of example only, to enhance the activity of the target, to inhibit the activity of the target, to limit the activity of the target, or to extend the activity of the target.

IC₅₀ as used herein refers to an amount, concentration or dosage of a particular test compound that achieves a 50% inhibition of a maximal effect, in an assay that measures such effect.

EC₅₀ as used herein refers to a dosage, concentration or amount of a test compound that elicits a dose-dependent response at 50% of maximal expression of a particular response that is induced, stimulated or potentiated by the particular test compound.

The GI₅₀ as used herein refers to the medicament concentration required to inhibit 50% of cell growth, i.e., the medicament concentration at which the growth of 50% of cells (such as cancer cells) is inhibited or controlled.

### Novel kinase inhibitor of the invention

The invention provides a compound of Formula (I), or a pharmaceutically acceptable salt, solvate, ester, acid, metabolite or prodrug thereof: 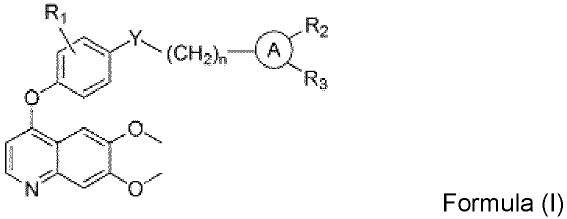
wherein, Y is selected from 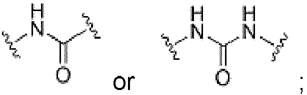
A is selected from aryl or 6-membered heterocyclyl;
n is an integer selected from 1-3;
R₁ is selected from hydrogen, halo, C₁₋₆alkyl, C₁₋₆haloalkyl, C₁₋₆alkoxy and cyano;
each of R₂ and R₃ is independently selected from hydrogen, halo, C₁₋₆alkyl, C₁₋₆haloalkyl, C₁₋₆alkoxy, or R₂ and R₃ together form a phenyl or 5-membered heterocyclyl.

In a preferred embodiment, n is preferably 1 when Y is 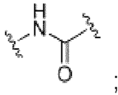 n is preferably 3 when Y is 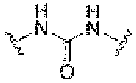 . In another aspect, A is preferably selected from phenyl, N-morpholinyl, N-piperidyl or N-piperazinyl. Further preferably, R₁ is selected from hydrogen, fluoro, chloro, methyl, ethyl, propyl, or cyano; each of R₂ and R₃ is independently selected from hydrogen, fluoro, chloro, methyl, ethyl, propyl, trifluoromethyl, difluoromethyl, trifluoroethyl, methoxy, ethoxy, propoxy, or R₂ and R₃ together form a phenyl or dioxolane.

In a preferred embodiment, the invention provides a compound of Formula (Ia), or a pharmaceutically acceptable salt, solvate, ester, acid, metabolite or prodrug thereof: 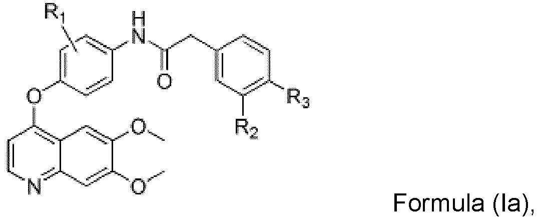 wherein R₁, R₂ and R₃ are defined as above.

More preferably, wherein R₁ is selected from hydrogen, fluoro, chloro, and methyl; each of R₂ and R₃ is independently selected from hydrogen, fluoro, chloro, methyl, and trifluoromethyl, or R₂ and R₃ together form a phenyl.

In a further preferred embodiment, the invention provides a compound of Formula (Ib), or a pharmaceutically acceptable salt, solvate, ester, acid, metabolite or prodrug thereof: 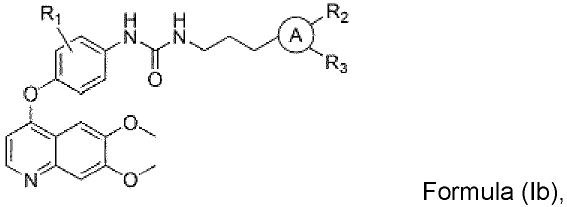 wherein A, R₁, R₂ and R₃ are defined as above.

More preferably, wherein A is selected from phenyl or N-morpholinyl; R₁ is hydrogen; each of R₂ and R₃ is independently selected from hydrogen, fluoro, chloro, methyl and trifluoromethyl.

In a preferred embodiment, the invention relates to compounds of Table 1 as below or pharmaceutically acceptable salts, solvates, esters, acids, metabolites or prodrugs thereof.

**Table 1**

| No. | Structure of Compound | No. | Structure of Compound |
|---|---|---|---|
| 1 | 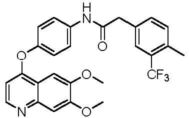 | 2 | 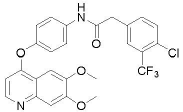 |
| 3 | 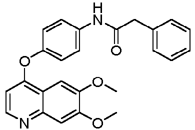 | 4 | 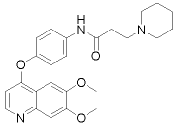 |
| 5 | 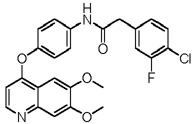 | 6 | 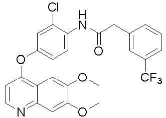 |
| 7 | 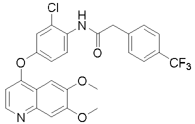 | 8 | 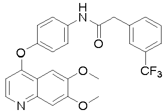 |
| 9 | 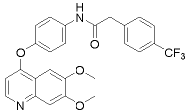 | 10 | 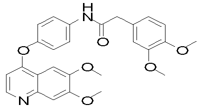 |
| 11 | 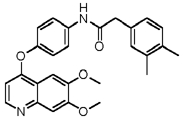 | 12 | 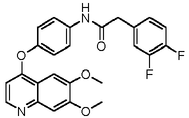 |
| 13 | 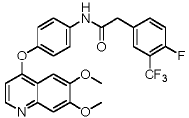 | 14 | 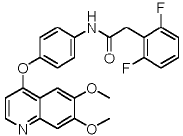 |
| 15 | 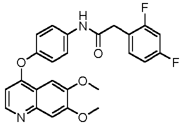 | 16 | 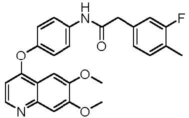 |
| 17 | 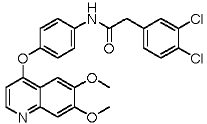 | 18 | 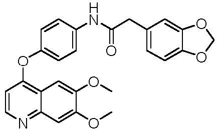 |
| 19 | 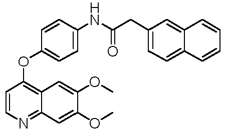 | 20 | 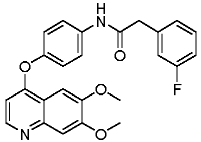 |
| 21 | 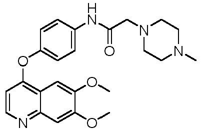 | 22 | 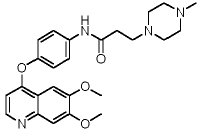 |
| 23 | 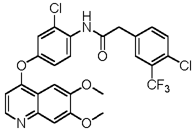 | 24 | 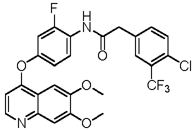 |
| 25 | 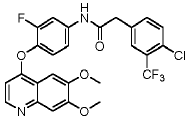 | 26 | 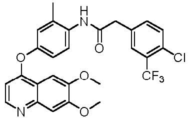 |
| 27 | 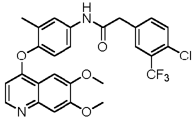 | 28 | 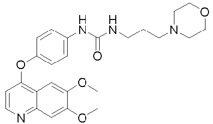 |
| 29 | 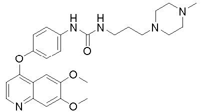 | 30 | 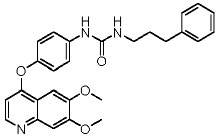 |
| 31 | 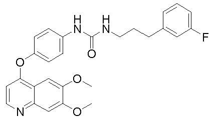 | 32 | 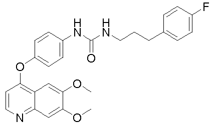 |
| 33 | 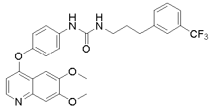 | 34 | 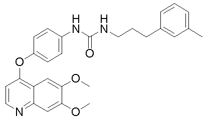 |
| 35 | 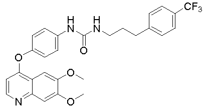 | 36 | 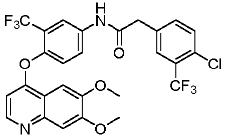 |
| 37 | 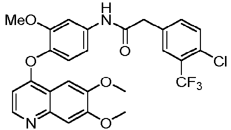 | 38 | 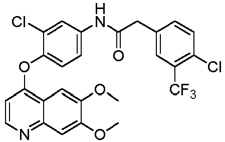 |
| 39 | 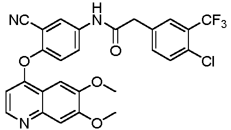 | 40 | 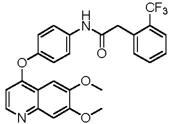 |

Any combination of the groups described above for the various variables is contemplated herein. It is understood that substituents and substitution patterns on the compounds provided herein can be selected by one of ordinary skill in the art to provide compounds that are chemically stable and that can be synthesized by techniques known in the art, as well as those set forth herein.

The pharmaceutically acceptable salts, solvates, esters, acids, pharmaceutically active metabolites and prodrugs of these compounds are also described herein.

In additional or further embodiments, the compounds described herein are metabolized upon administration to an organism in need to produce a metabolite that is then used to produce a desired effect, including a desired therapeutic effect.

Compounds described herein may be formed as, and/or used as, pharmaceutically acceptable salts. The type of pharmaceutical acceptable salts, include, but are not limited to: (1) acid-addition salts, formed by reacting the free base form of the compound with a pharmaceutically acceptable inorganic acid such as hydrochloric acid, hydrobromic acid, sulfuric acid, nitric acid, phosphoric acid, metaphosphoric acid, and the like; or with an organic acid such as acetic acid, propionic acid, hexanoic acid, cyclopentane propionic acid, glycolic acid, pyruvic acid, lactic acid, malonic acid, malic acid, citric acid, succinic acid, maleic acid, tartaric acid, fumaric acid, trifluoroacetic acid, benzoic acid, 3-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, toluenesulfonic acid, 4-methylbicyclo-[2.2.2]oct-2-ene-1-carboxylic acid, 2-naphthalenesulfonic acid, tert-butylacetic acid, glucoheptonic acid, 4,4'-methylenebis-(3-hydroxy-2-ene-1-carboxylic acid), 3-phenylpropionic acid, trimethylacetic acid, lauryl sulfuric acid, gluconic acid, glutamic acid, salicylic acid, hydroxynaphthoic acid, stearic acid, muconic acid, and the like; (2) base-addition salts formed when an acidic proton present in the parent compound either is replaced by a metal ion, e.g., an alkali metal ion (e.g. lithium, sodium, potassium), an alkaline earth metal ion (e.g. magnesium, or calcium), or an aluminum ion; or coordinates with an organic base or an inorganic base, and acceptable organic bases include ethanolamine, diethanolamine, triethanolamine, trimethylamine, N-methylglucamine, and the like, and acceptable inorganic bases include aluminum hydroxide, calcium hydroxide, potassium hydroxide, sodium carbonate, sodium hydroxide, and the like.

The corresponding counterions of the pharmaceutically acceptable salts may be analyzed and identified using various methods including, but not limited to, ion exchange chromatography, ion chromatography, capillary electrophoresis, inductively coupled plasma, atomic absorption spectroscopy, mass spectrometry, or any combination thereof.

The salts are recovered by using at least one of the following techniques: filtration, precipitation with a non-solvent followed by filtration, evaporation of the solvent, or, in the case of aqueous solutions, lyophilization.

The screening and characterization of the pharmaceutically acceptable salts, polymorphs and/or solvates may be accomplished using a variety of techniques including, but not limited to, thermal analysis, x-ray diffraction, spectroscopy, microscopy, and elemental analysis. The various spectroscopic techniques used include, but are not limited to, Raman, FTIR, UVIS, and NMR (liquid and solid state). The various microscopy techniques include, but are not limited to, IR microscopy and Raman microscopy.

### Pharmaceutical use of the invention

The compounds of Formula (I), Formula (Ia) and Formula (Ib) of the invention, or pharmaceutically acceptable salts, solvates, esters, acids, metabolites or prodrugs thereof, can transform macrophages in TME from M2 that promotes tumor growth to M1, thereby changing TME and achieving the purpose of treating cancer. In addition, the compounds of Formula (I), Formula (Ia) and Formula (Ib) of the invention, or pharmaceutically acceptable salts, solvates, esters, acids, metabolites or prodrugs thereof, can inhibit the activity of CSF1R kinase and related signaling pathways, thereby achieving the purpose of treating cancer.

Therefore, the compounds of Formula (I), Formula (Ia) and Formula (Ib) of the invention, or pharmaceutically acceptable salts, solvates, esters, acids, metabolites or prodrugs thereof, can be used to treat cancers or tumors in which M2 macrophages are dominant in the tumor microenvironment.

The cancer or tumor is selected from one or more of ovarian cancer, cervical cancer, membranous adenocarcinoma, prostate cancer, bladder cancer, lung cancer, thyroid cancer, breast cancer, pancreatic cancer, kidney cancer, gastric cancer, liver cancer, cervix cancer, endometrial cancer, colorectal cancer, nasopharyngeal cancer, esophageal cancer, bile duct cancer, bone metastatic cancer, papillary thyroid cancer, non-small cell lung cancer, small cell lung cancer, colon cancer, solid tumor, brain tumor, lymphoma, glioma, glioblastoma, melanoma, mesothelioma, glioblastoma, osteosarcoma, gastrointestinal stromal tumor, multiple myeloma, carcinosarcoma of the bile duct, and leukemia. Of these, the lymphoma comprises non-Hodgkin's lymphoma, B cell or T cell lymphoma; the leukemia comprises chronic myelocytic leukemia or acute myeloid leukemia.

In an embodiment of the invention, a medicament comprising the compound of the invention may be administered to a patient by at least one of injection, oral administration, inhalation, rectal administration, and transdermal administration. When a patient is treated in accordance with the invention, the amount of a given agent depends on many factors, such as the specific dosing regimen, the type of the disease or condition and its severity, the identity (e.g., weight) of the subject or host in need of treatment, whereas the administered dose can be routinely determined in a manner known in the art according to the particular circumstances surrounding the case, including, e.g., the specific agent being administered, the route of administration, the condition being treated, and the subject or host being treated. In general, doses employed for adult human treatment will typically be in the range of 0.02-5000 mg per day, such as from about 1-1500 mg per day. The desired dose may conveniently be presented in a single dose or as divided doses administered simultaneously (or over a short period of time) or at appropriate intervals, for example as two, three, four or more divided doses per day. It will be appreciated by those skilled in the art that, although the above dosage ranges are given, the specific effective amounts may be appropriately adjusted depending on the condition of the patient and the judgment of the practitioner.

### Preparation of the compounds

Compounds of Formula (I) may be synthesized using standard synthetic techniques known to those of skill in the art or using methods known in the art in combination with methods described herein. In additions, solvents, temperatures and other reaction conditions presented herein may vary according to those of skill in the art. As a further guide, the following synthetic methods may also be utilized.

The reactions can be employed sequentially to provide the compounds described herein; or they may be used to synthesize fragments which are subsequently joined by the methods described herein and/or known in the art.

In certain embodiments, provided herein are methods of preparing the tyrosine kinase inhibitor compounds described herein and methods of using them. In certain embodiments, compounds described herein can be synthesized using the following synthetic schemes. The compounds may be synthesized using methods analogous to those described below by the use of appropriate alternative starting materials.

The starring materials used for the synthesis of the compounds described herein may be synthesized or can be obtained from commercial sources. The compounds described herein and other related compounds having different substituents can be synthesized using techniques and materials known to those of skill in the art. General methods for the preparation of compounds as disclosed herein may be derived from known reactions in the field, and the reactions may be modified by the use of appropriate reagents and conditions, as would be recognized by the skilled person, for the introduction of the various moieties into the molecules as provided herein.

The products of the reactions may be isolated and purified, if desired, using conventional techniques, including, but not limited to, filtration, distillation, crystallization, chromatography, and the like. Such products may be characterized using conventional means, including physical constants and spectral data.

A non-limiting example of the synthetic scheme for the preparation of a compound of Formula (I) is shown in the following synthetic route.

### Example 1: N-(4-((6,7-dimethoxyquinolin-4-yl)oxy)phenyl)-2-(4-methyl-3-(trifluoromethyl)phenyl)acetamide

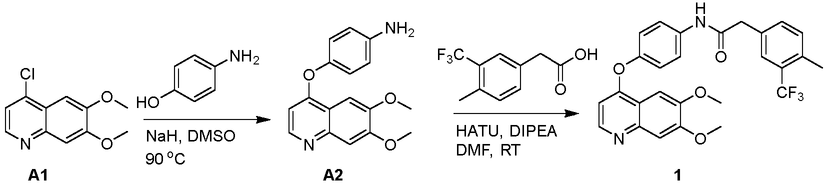

4-((6,7-dimethoxyquinolin-4-yl)oxy)phenylamine (A2): to a round-bottom flask was added 4-hydroxyphenylamine (2.0 g), followed by dimethyl sulfoxide (30 ml), and then by addition of sodium hydride (807 mg) dropwise under the condition of ice bath. The reaction system was stirred for 30 min at room temperature. Next, 4-chloro-6,7-dimethoxyquinoline (4.1 g) was added, and the reaction system was allowed to react overnight at 90°C. After completion of the reaction, the system was added with water and filtered, with the solids subjected to washing with water and a small amount of methanol. The resultant brown solids were used directly in the next step.

N-(4-((6,7-dimethoxyquinolin-4-yl)oxy)phenyl)-2-(4-methyl-3-(trifluoromet hyl)phenyl)acetamide (1): to a round-bottom flask was added 4-((6,7-dimethoxyquinolin-4-yl)oxy)phenylamine (1 g), followed by N,N-dimethylformamide (10 ml), HATU (1.92 g), 4-methyl-3-trifluoromethylphenylacetic acid (1.10 g) and N,N-diisopropylethylamine (0.87 g). The reaction system was stirred overnight at room temperature. After completion of the reaction, the system was added with water and extracted with ethyl acetate, with the organic phase subjected to drying over anhydrous sodium sulfate. The organic phase was filtered, the solvent was removed from the filtrate under reduced pressure, and the residue was purified via pressurized silica gel column chromatography to obtain the Compound **1.** LC/MS: M+H 497.1.

### Example 2: N-(4-((6,7-dimethoxyauinolin-4-yl)oxy)phenyl)-2-(4-chloro-3-(trifluoromethyl)ph enyl)-acetamide

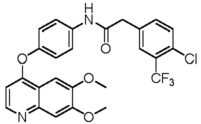

Synthesis of the compound of Example 2 was completed by using procedures similar to those of Example 1. MS(ESI) m/z(M+1)+: 517.11.

### Example 3: N-(4-((6,7-dimethoxyauinolin-4-yl)oxy)phenyl)-2-phenylacetamide

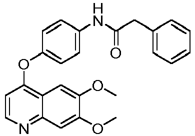

Synthesis of the compound of Example 3 was completed by using procedures similar to those of Example 1. MS(ESI) m/z(M+1)+: 415.16.

### Example 4: N-(4-((6, 7-dimethoxyauinolin-4-yl)oxy)phenyl)-3-(piperidin-1-yl)propanam ide

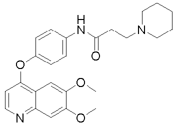

Synthesis of the compound of Example 4 was completed by using procedures similar to those of Example 1. MS(ESI) m/z(M+1)+: 436.22.

### Example 5: N-(4-((6,7-dimethoxyauinolin-4-yl)oxy)phenyl)-2-(4-chloro-3-fluorophenyl)acet amide

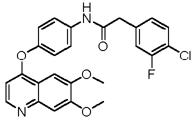

Synthesis of the compound of Example 5 was completed by using procedures similar to those of Example 1. MS(ESI) m/z(M+1)+: 467.11.

### Example 6: N-(2-chloro-4-((6,7-dimethoxyquinolin-4-yl)oxy)phenyl)-2-(3-(trifluoromethyl)ph enyl)acetamide

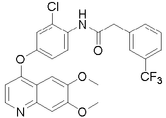

Synthesis of the compound of Example 6 was completed by using procedures similar to those of Example 1. MS(ESI) m/z(M+1)+: 517.11.

### Example 7: N-(2-chloro-4-((6,7-dimethoxyquinolin-4-yl)oxy)phenyl)-2-(4-(trifluoromethyl)ph enyl)acetamide

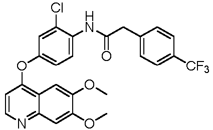

Synthesis of the compound of Example 7 was completed by using procedures similar to those of Example 1. MS(ESI) m/z(M+1)+: 517.11.

### Example 8: N-(4-((6,7-dimethoxyauinolin-4-yl)oxy)phenyl)-2-(3-(trifluoromethyl)phenyl)ace tamide

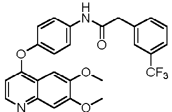

Synthesis of the compound of Example 8 was completed by using procedures similar to those of Example 1. MS(ESI) m/z(M+1)+: 483.15.

### Example 9: N-(4-((6,7-dimethoxyquinolin-4-yl)oxy)phenyl)-2-(4-(trifluoromethyl)phenyl)ace tamide

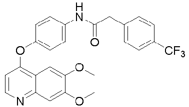

Synthesis of the compound of Example 9 was completed by using procedures similar to those of Example 1. MS(ESI) m/z(M+1)+: 483.15.

### Example 10: N-(4-((6,7-dimethoxyquinolin-4-yl)oxy)phenyl)-2-(3,4-dimethoxyphenyl)acetam ide

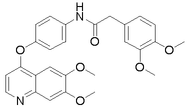

Synthesis of the compound of Example 10 was completed by using procedures similar to those of Example 1. MS(ESI) m/z(M+1)+: 475.18.

### Example 11: N-(4-((6,7-dimethoxyquinolin-4-yl)oxy)phenyl)2-(3,4-dimethylphenyl)acetamid e

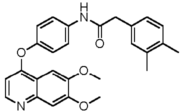

Synthesis of the compound of Example 11 was completed by using procedures similar to those of Example 1. MS(ESI) m/z(M+1)+: 443.19.

### Example 12: N-(4-((6,7-dimethoxyquinolin-4-yl)oxy)phenyl)-2-(3,4-difluorophenyl)acetamide

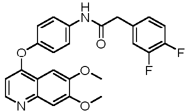

Synthesis of the compound of Example 12 was completed by using procedures similar to those of Example 1. MS(ESI) m/z(M+1)+: 451.14.

### Example 13: N-(4-((6,7-dimethoxyquinolin-4-yl)oxy)phenyl)-2-(4-fluoro-3-(trifluoromethyl)ph enyl)acetamide

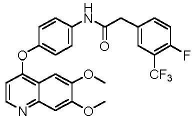

Synthesis of the compound of Example 13 was completed by using procedures similar to those of Example 1. MS(ESI) m/z(M+1)+: 501.14.

### Example 14: N-(4-((6,7-dimethoxyquinolin-4-yl)oxy)phenyl)-2-(2,6-difluorophenyl)acetamide

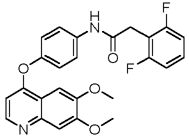

Synthesis of the compound of Example 14 was completed by using procedures similar to those of Example 1. MS(ESI) m/z(M+1)+: 451.14.

### Example 15: N-(4-((6,7-dimethoxyquinolin-4-yl)oxy)phenyl)-2-(2,4-difluorophenyl)acetamide

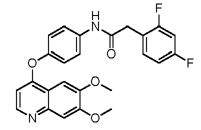

Synthesis of the compound of Example 15 was completed by using procedures similar to those of Example 1. MS(ESI) m/z(M+1)+: 451.14.

### Example 16: N-(4-((6,7-dimethoxyquinolin-4-yl)oxy)phenyl)-2-(3-fluoro-4-methylphenyl)acet amide

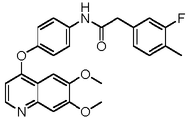

Synthesis of the compound of Example 16 was completed by using procedures similar to those of Example 1. MS(ESI) m/z(M+1)+: 447.17.

### Example 17: N-(4-((6,7-dimethoxyauinolin-4-yl)oxy)phenyl)-2-(3,4-dichlorophenyl)acetamid e

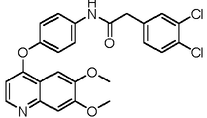

Synthesis of the compound of Example 17 was completed by using procedures similar to those of Example 1. MS(ESI) m/z(M+1)+: 483.08.

### Example 18: N-(4-((6,7-dimethoxyquinolin-4-yl)oxy)phenyl)-2-(benzo[d][1,3]dioxol-5-yl)acet amide

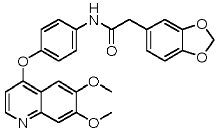

Synthesis of the compound of Example 18 was completed by using procedures similar to those of Example 1. MS(ESI) m/z(M+1)+: 459.15.

### Example 19: N-(4-((6,7-dimethoxyquinolin-4-yl)oxy)phenyl)-2-(naphth-2-yl)acetamide

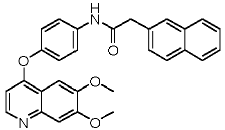

Synthesis of the compound of Example 19 was completed by using procedures similar to those of Example 1. MS(ESI) m/z(M+1)+: 465.18.

### Example 20: N-(4-((6,7-dimethoxyquinolin-4-yl)oxy)phenyl)-2-(3-fluorophenyl)acetamide

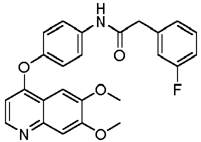

Synthesis of the compound of Example 20 was completed by using procedures similar to those of Example 1. MS(ESI) m/z(M+1)+: 433.15.

### Example 21: N-(4-((6,7-dimethoxyquinolin-4-yl)oxy)phenyl)-2-(4-methylpiperazin-1-yl)aceta mide

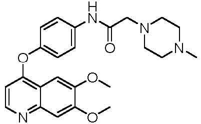

Synthesis of the compound of Example 21 was completed by using procedures similar to those of Example 1. MS(ESI) m/z(M+1)+: 437.21.

### Example 22: N-(4-((6,7-dimethoxyquinolin-4-yl)oxy)phenyl)-3-(4-methylpiperazin-1-yl)propa namide

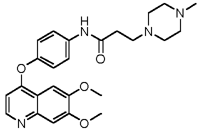

Synthesis of the compound of Example 22 was completed by using procedures similar to those of Example 1. MS(ESI) m/z(M+1)+: 451.23.

### Example 23: N-(2-chloro-4-((6,7-dimethoxyquinolin-4-yl)oxy)phenyl)-2-(4-chloro-3-(trifluoro methyl)phenyl)acetamide

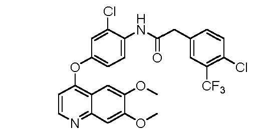

Synthesis of the compound of Example 23 was completed by using procedures similar to those of Example 1. MS(ESI) m/z(M+1)+: 551.07.

### Example 24: N-(2-fluoro-4-((6,7-dimethoxyquinolin-4-yl)oxy)phenyl)-2-(4-chloro-3-(trifluoro methyl)phenyl)acetamide

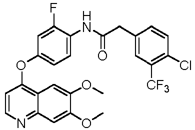

Synthesis of the compound of Example 24 was completed by using procedures similar to those of Example 1. MS(ESI) m/z(M+1)+: 535.10.

### Example 25: N-(3-fluoro-4-((6,7-dimethoxyquinolin-4-yl)oxy)phenyl)-2-(4-chloro-3-(trifluoro methyl)phenyl)acetamide

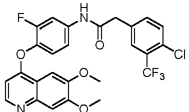

Synthesis of the compound of Example 25 was completed by using procedures similar to those of Example 1. MS(ESI) m/z(M+1)+: 535.10.

### Example 26: N-(2-methyl-4-((6,7-dimethoxyauinolin-4-yl)oxy)phenyl)-2-(4-chloro-3-(trifluoro methyl)phenyl)acetamide

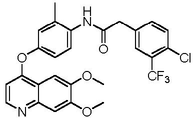

Synthesis of the compound of Example 26 was completed by using procedures similar to those of Example 1. MS(ESI) m/z(M+1)+: 531.12.

### Example 27: N-(3-methyl-4-((6,7-dimethoxyauinolin-4-yl)oxy)phenyl)-2-(4-chloro-3-(trifluoro methyl)phenyl)acetamide

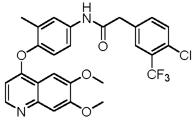

Synthesis of the compound of Example 27 was completed by using procedures similar to those of Example 1. MS(ESI) m/z(M+1)+: 531.12.

### Example 28: 1-(4-((6,7-dimethoxyauinolin-4-yl)oxy)phenyl)-3-(3-morpholinopropyl)urea

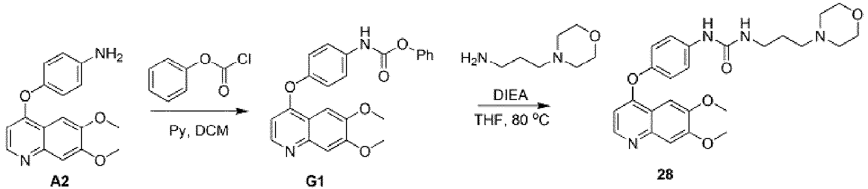

Phenyl (4-((6,7-dimethoxyquinolin-4-yl)oxy)phenyl)carbamate (G1): 4-((6,7-dimethoxyquinolin-4-yl)oxy)phenylamine (1 g) was dissolved in dichloromethane (30 ml), followed by addition of pyridine (0.4 g), and by dropwise addition of phenyl chloroformate (0.8 g) under the condition of ice bath. Stirring was continued for 3 h after the addition. After completion of the reaction, the reaction system was added with water and extracted with dichloromethane. The organic phase was dried over anhydrous sodium sulfate and subjected to filtering. Dichloromethane was removed under reduced pressure and the residue was purified via pressurized silica gel column chromatography to obtain the Compound G1. MS: (M+1) 417.14.

1-(4-((6,7-dimethoxyquinolin-4-yl)oxy)phenyl)-3-(3-morpholinopropyl)urea (42): to a round-bottom flask was added phenyl (4-((6,7-dimethoxyquinolin-4-yl)oxy)phenyl) carbamate (50 mg), N-(3-aminopropyl)morpholine (26 mg), N,N-diisopropylethylamine (23 mg) and tetrahydrofuran (3 ml). The reaction mixture was allowed to react overnight at 80°C. After completion of the reaction, the reaction system was concentrated and the residue was purified via pressurized silica gel column chromatography to obtain Compound 28. MS: (M+1) 467.22.

### Example 29: 1-(4-((6,7-dimethoxyauinolin-4-yl)oxy)phenyl)-3-(3-(4-methylpiperazin-1-yl)pro pyl)urea

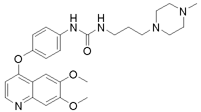

Synthesis of the compound of Example 29 was completed by using procedures similar to those of Example 28. MS(ESI) m/z(M+1)+: 480.26.

### Example 30: 1-(4-((6,7-dimethoxyauinolin-4-yl)oxy)phenyl)-3-(3-phenylpropyl)urea

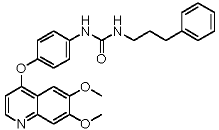

Synthesis of the compound of Example 30 was completed by using procedures similar to those of Example 28. MS(ESI) m/z(M+1)+: 458.20.

### Example 31: 1-(4-((6,7-dimethoxyauinolin-4-yl)oxy)phenyl)-3-(3-(3-fluorophenyl)propyl)urea

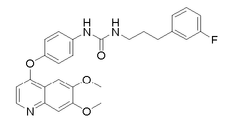

Synthesis of the compound of Example 31 was completed by using procedures similar to those of Example 28. MS(ESI) m/z(M+1)+: 476.19.

### Example 31: 1-(4-((6,7-dimethoxyquinolin-4-yl)oxy)phenyl)-3-(3-(4-fluorophenyl)propyl)urea

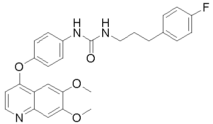

Synthesis of the compound of Example 32 was completed by using procedures similar to those of Example 28. MS(ESI) m/z(M+1)+: 476.19.

### Example 33: 1-(4-((6,7-dimethoxyquinolin-4-yl)oxy)phenyl)-3-(3-(3-(trifluoromethyl)phenyl)p ropyl)urea

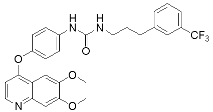

Synthesis of the compound of Example 33 was completed by using procedures similar to those of Example 28. MS(ESI) m/z(M+1)+: 526.19.

### Example 34: (1-(4-((6,7-dimethoxyquinolin-4-yl)oxy)phenyl)-3-(3-(3-(methyl)phenyl)propyl)u rea

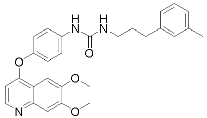

Synthesis of the compound of Example 34 was completed by using procedures similar to those of Example 28. MS(ESI) m/z(M+1)+: 472.22.

### Example 35: 1-(4-((6,7-dimethoxyquinolin-4-yl)oxy)phenyl)-3-(3-(4-(trifluoromethyl)phenyl)p ropyl)urea

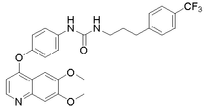

Synthesis of the compound of Example 35 was completed by using procedures similar to those of Example 28. MS(ESI) m/z(M+1)+: 526.19.

### Example 36: N-(3-trifluoromethyl-4-((6,7-dimethoxyquinolin-4-yl)oxy)phenyl)-2-(4-chloro-3-( trifluoromethyl)phenyl)acetamide

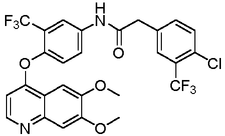

Synthesis of the compound of Example 36 was completed by using procedures similar to those of Example 1. MS(ESI) m/z(M+1)+: 585.10.

### Example 37: N-(3-methoxy-4-((6,7-dimethoxyquinolin-4-yl)oxy)phenyl)-2-(4-chloro-3-(trifluo romethyl)phenyl)acetamide

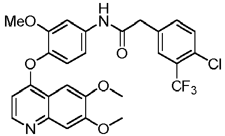

Synthesis of the compound of Example 37 was completed by using procedures similar to those of Example 1. MS(ESI) m/z(M+1)+: 547.12.

### Example 38: N-(3-chloro-4-((6,7-dimethoxyquinolin-4-yl)oxy)phenyl)-2-(4-chloro-3-(trifluoro methyl)phenyl)acetamide

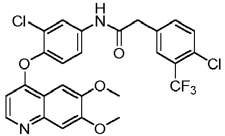

Synthesis of the compound of Example 38 was completed by using procedures similar to those of Example 1. MS(ESI) m/z(M+1)+: 551.08.

### Example 39: N-(3-cyano-4-((6,7-dimethoxyquinolin-4-yl)oxy)phenyl)-2-(4-chloro-3-(trifluoro methyl)phenyl)acetamide

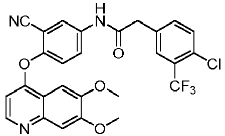

Synthesis of the compound of Example 39 was completed by using procedures similar to those of Example 1. MS(ESI) m/z(M+1)+: 542.11.

### Example 40: N-(4-((6,7-dimethoxyquinolin-4-yl)oxy)phenyl)-2-(2-(trifluoromethyl)phenyl)ace tamide

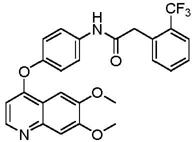

Synthesis of the compound of Example 40 was completed by using procedures similar to those of Example 1. MS(ESI) m/z(M+1)+: 483.15.

### Comparative Example 1: N-(6-((6,7-dimethoxyauinolin-4-yl)oxy)pyridin-3-yl)-2-phenylacetamide

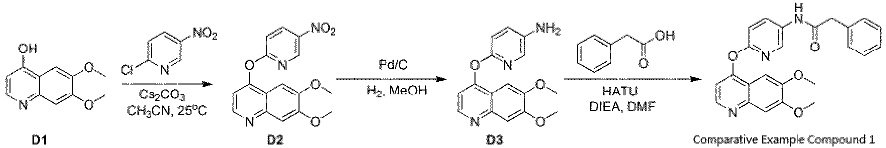

6,7-dimethoxy-4-((5-nitropyridin-2-yl)oxy)quinoline (D2): to a round-bottom flask was added 4-hydroxy-6,7-dimethoxyquinoline (0.5 g), followed by addition of acetonitrile (10 ml), and then by cesium carbonate (0.87 g). The mixture was stirred at room temperature for 5 minutes. Then 2-chloro-5-nitropyridine (0.42 g) was added and stirring was conducted overnight at room temperature. Filtering was conducted after completion of the reaction, the filtrate was concentrated and the residue was purified via pressurized silica gel column chromatography to obtain Compound D2.

6-((6,7-dimethoxyquinolin-4-yl)oxy)pyridin-3-amine (D3): to a round-bottom flask was added 6,7-dimethoxy-4-((5-nitropyridin-2-yl)oxy)quinoline (0.42 g), and followed by addition of methanol (10 ml), palladium/carbon (0.11 g). Reaction was allowed to occur overnight under hydrogen atmosphere. Filtering was conducted after completion of the reaction, the filtrate was concentrated and the residue was purified via pressurized silica gel column chromatography to obtain Compound D3. LC/MS: M+H 298.11.

N-(6-((6,7-dimethoxyquinolin-4-yl)oxy)pyridin-3-yl)-2-phenylacetamide (Comparative Example Compound 1): to a round-bottom flask was added 6-((6,7-dimethoxyquinolin-4-yl)oxy)pyridin-3-amine (50 mg), followed by addition of N,N-dimethylformamide (2 ml), HATU (96 mg), phenylacetic acid (34 mg) and N,N-diisopropylethylamine (66 mg). The reaction system was stirred overnight at room temperature. After completion of the reaction, the system was added with water and extracted with ethyl acetate, with the organic phase subjected to drying over anhydrous sodium sulfate. The organic phase was filtered, the solvent was removed from the filtrate under reduced pressure, and the residue was purified via pressurized silica gel column chromatography to obtain Comparative Example Compound 1. LC/MS: M+H 416.16.

### Comparative Example 2: N-(5-chloro-6-((6,7-dimethoxyauinolin-4-yl)oxy)pyridin-3-yl)-2-(3-(trifluorometh yl)phenyl)acetamide

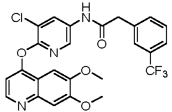

Synthesis of the compound of Comparative Example 2 was completed by using procedures similar to those of Comparative Example 1. MS(ESI) m/z(M+1)+: 518.10.

### Comparative Example 3: N-(3-(trifluoromethyl)benzyl)-4-((6,7-dimethoxyauinolin-4-yl)oxy)benzamide

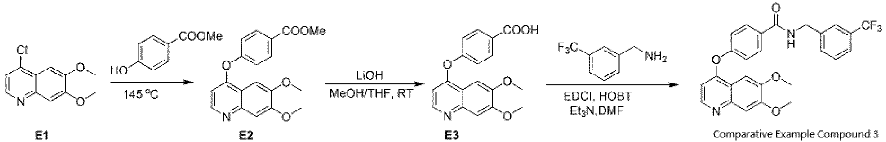

Methyl 4-((6,7-dimethoxyquinolin-4-yl)oxy)benzoate (E2): to a round-bottom flask was added 4-chloro-6,7-dimethoxyquinoline (1.0 g) and methyl 4-hydroxybenzoate (0.68 g), and the mixture was stirred overnight at 145°C. After completion of the reaction, the mixture was added with saturated sodium bicarbonate solution, extracted with ethyl acetate, and the organic phase was dried over anhydrous sodium sulfate and filtered. The filtrate was concentrated and the residue was purified via pressurized silica gel column chromatography to obtain Compound E2.

4-((6,7-dimethoxyquinolin-4-yl)oxy)benzoic acid (E3): to a round-bottom flask was added methyl 4-((6,7-dimethoxyquinolin-4-yl)oxy)benzoate (0.5 g), methanol/ tetrahydrofuran (5:1, 10 ml), and lithium hydroxide (0.18 g). Reaction was allowed to occur overnight at room temperature. Concentration was conducted after completion of the reaction, and pH was adjusted to about 5 with concentrated hydrochloric acid. Filtering was conducted and the filter cake was washed with water to obtain the Compound E3. LC/MS: M+H 326.10.

N-(3-(trifluoromethyl)benzyl)-4-((6,7-dimethoxyquinolin-4-yl)oxy)benzamid e (Comparative Example Compound 3): to a round-bottom flask was added 4-((6,7-dimethoxyquinolin-4-yl)oxy)benzoic acid (20 mg), and followed by addition of N,N-dimethylformamide (2 ml), 3-trifluoromethylbenzylamine (13 mg), EDCI (17 mg), HOBT (12 mg) and triethylamine (12 mg). The reaction system was stirred overnight at room temperature. After completion of the reaction, the system was added with water and extracted with ethyl acetate, with the organic phase subjected to drying over anhydrous sodium sulfate. The organic phase was filtered and the solvent was removed from the filtrate under reduced pressure. The residue was purified via pressurized silica gel column chromatography to obtain Comparative Example Compound 3. LC/MS: M+H 483.15.

### Comparative Example 4: N-(4-((6,7-dimethoxyquinazolin-4-yl)oxy)phenyl)-2-(3-methoxyphenyl)acetami de

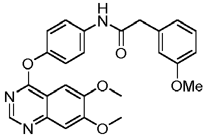

Synthesis of the compound of Comparative Example 4 was completed by using procedures similar to those of Example 1. MS(ESI) m/z(M+1)+: 446.17.

### Example 41: Effects on proliferation of cancer cells

The inhibitory effect of the compounds provided herein on proliferation of cancer cells was further evaluated by testing the effect of the compounds of the invention on growth of cancer cells.

In the example, different concentrations (0.000508 µM, 0.00152 µM, 0.00457 µM, 0.0137 µM, 0.0411 µM, 0.123 µM, 0.370 µM, 1.11 µM, 3.33 µM, 10 µM in DMSO) of the compounds of the invention and Comparative Example Compounds 1-4 were added to TEL-CSF1R-BaF3 (constructed in our laboratory) and M-NSF-60 (purchased from COBIOER BIOSCIENCES CO., LTD, Nanjing, China, a M-CSF (CSF-1)-mediated mouse myeloblast cell line, when the inhibitory effect of a compound on cancer cell proliferation is evaluated using this cell line, M-CSF at a concentration of 50 ng/mL is added to the cell culture medium), and incubated for 72 hours. The incubated cells were detected by CCK-8 (purchased from MedChem Express, China) cell viability detection kit (CCK-8 can be reduced by dehydrogenases in living cells to a highly water-soluble yellow formazan product, and the amount of the generated formazan product is proportional to the number of living cells). The number of living cells was quantified by a microplate reader, and the GI₅₀ for each compound was calculated (the results are shown in Table 2).

The construction method of the TEL-CSF1R-BaF3 cell line (a cell line stably expressing CSF1R kinase) was as follows: the sequence of human CSF1R kinase region was amplified by PCR and inserted into the MSCV-Puro vector (Clontech) with an N-terminal TEL fragment, and stably transferred into mouse BaF3 cells (purchased from ATCC, USA) by a retroviral method, and IL-3 growth factor was removed, finally a cell line dependent on CSF1R transferred protein was obtained.

The results in Table 2 showed that the compounds of the invention had a strong inhibitory effect on CSF1R kinase activity. Comparative Example Compounds 1-3 had no inhibitory effect on the proliferation of TEL-CSF1R-BaF3, which expressed the CSF1R kinase, and M-NSF-60, a M-CSF-mediated mouse myeloblast cell line. Comparative Example Compound 4 had certain inhibitory activity on the proliferation of TEL-CSF1R-BaF3, which expressed the CSF1R kinase, and M-NSF-60, a M-CSF-mediated mouse myeloblast cell line, but the activity was lower than that of the compounds of the invention. The compounds of the invention had a strong inhibitory effect on the proliferation of TEL-CSF1R-BaF3, which expressed the CSF1R kinase, and M-NSF-60, a M-CSF-mediated mouse myeloblast cell line. Since CSF1R kinase is a key protein that controls macrophage polarization in the tumor microenvironment, inhibiting the activity of CSF1R kinase can reduce the transformation of macrophages into M2 macrophages that promote cancer development, and increase the transformation into M1 macrophages that inhibit cancer development.

**Table 2. Inhibitory activity of the compounds of the invention on the proliferation of different cancer cells**

| Example No. | TEL-CSF1R-BaF3 (GI₅₀/µM) | M-NSF-60 (Gl₅₀/µM) |
|---|---|---|
| 1 | 0.002 | 0.009 |
| 2 | 0.003 | 0.03 |
| 3 | 0.012 | 0.1 |
| 5 | 0.001 | 0.035 |
| 6 | 0.017 | 0.06 |
| 7 | 0.009 | 0.03 |
| 8 | 0.001 | 0.006 |
| 9 | 0.002 | 0.007 |
| 10 | 0.009 | 0.038 |
| 11 | 0.001 | 0.005 |
| 12 | 0.003 | 0.065 |
| 13 | 0.002 | 0.018 |
| 14 | 0.012 | 0.26 |
| 15 | 0.003 | 0.051 |
| 16 | 0.001 | 0.033 |
| 17 | 0.002 | 0.042 |
| 18 | 0.002 | 0.062 |
| 19 | 0.0008 | 0.074 |
| 20 | 0.006 | 0.13 |
| 23 | 0.008 | 0.061 |
| 24 | 0.002 | 0.024 |
| 25 | 0.003 | 0.05 |
| 26 | 0.006 | 0.06 |
| 27 | 0.003 | 0.041 |
| 28 | 0.012 | -- |
| 29 | 0.011 | -- |
| 37 | 0.004 | 0.075 |
| 38 | 0.026 | 0.32 |
| 39 | 0.022 | 0.61 |
| 40 | 0.007 | 0.091 |
| Comparative Example 1 | >10 | >10 |
| Comparative Example 2 | 2.54 | 7.84 |
| Comparative Example 3 | 1.23 | 2.4 |
| Comparative Example 4 | 0.17 | 0.55 |

| | | |
|---|---|---|
| Note: "--" in Table 2 means not detected. | | |

### Example 42: Cell transformation experiment

RAW264.7 cells (purchased from ATCC, USA) were sucked up and down with a pipette, and the cells were added to a centrifuge tube, placed in a centrifuge, and centrifuged at 800 rpm for 4 minutes. The cell supernatant was discarded, and the cells were plated in a 6-well transparent plate at a density of 0.5×10⁵ cells/ml, and cultured with 1640 culture medium containing 10% FBS (1640 culture medium was purchased from Corning, USA, and FBS was purchased from Excel, Australia) in a 37°C, 5% CO₂ incubator for 12 hours; then the 6-well transparent plate was taken out, and 20 ng/ml interleukin-4 or interleukin-13 factor (both purchased from Sino Biological, Beijing, China) was added to the 1640 culture medium (purchased from Corning, USA), which were then added together into the cell wells of the 6-well transparent plate to culture the cells in the culture medium for 24 hours; cell morphology was checked, and if the cells gradually changed from round to spindle-shaped, secretion of interleukin-10 in the supernatant of the culture medium was detected using a mouse IL-10 ELISA kit (purchased from Wuhan Saipei Biotechnology, China) according to its instructions, and an increase of the concentration of interleukin-10 secretion suggested that RAW264.7 cells were induced into M2 macrophages. After the cells were successfully induced, they were treated with the randomly selected Compound 2 of the invention, the control compound BLZ945 and PLX3397 (purchased from MedChem Express, China), which have been diluted in series, for 72 hours, respectively, and then the cells and supernatant were collected for qPCR and ELISA experiments, respectively.

### qPCR-Extraction of Cell RNA

In this experiment, an RNA extraction kit (purchased from Tiangen Biochemical Technology, China, catalog number: DP451) was used. RNA extraction was performed according to the instructions, and the concentration of RNA in the sample was detected using a NanoDrop nucleic acid concentration meter, and the RNA solution was diluted to the same concentration.

### Real-time fluorescence quantitative PCR experiment

In this experiment, TaKaRa One Step TB Green^{®} PrimeScript^{™} PLUS RT-PCR Kit (purchased from TaKaRa Bio, catalog number: RR096A) was used, which contained the following components:

| **Reagent** | **Amount** |
|---|---|
| 2× One Step TB Green RT-PCR Buffer | 840 µL×3 |
| PrimeScript PLUS RTase Mix | 100 µL |
| TaKaRa Ex Taq HS Mix | 300 µL |
| ROX Reference Dye | 100 µL |
| ROX Reference Dye II | 100 µL |

The procedure was as follows:
① The PCR primers were taken out and placed on ice. The primer sequences used were as follows:

| **Primer** | **Forward primer** | **Reverse primer** |
|---|---|---|
| CD86 | TCCAAGTTTTTGGGCAATGTC | CCTATGAGTGTGCACTGAGTTAAACA |
| GAPDH | AAATGGTGAAGGTCGGTGTG | ATGAAGGGGTCGTTGATGGC |

② The RT-PCR reaction solution was prepared on ice as follows.

| **Reagent** | **Usage amount (per sample)** |
|---|---|
| 2× One Step TB Green RT-PCR Buffer | 10 µL |
| TaKaRa Ex Taq HS Mix | 1.2 µL |

| **Reagent** | **Usage amount (per sample)** |
|---|---|
| PrimeScript PLUS RTase Mix | 0.4 µL |
| PCR Forward Primer (10 µM) | 0.8 µL |
| PCR Reverse Primer (10 µM) | 0.8 µL |
| RNase Free dH₂O | 4.8 µL |

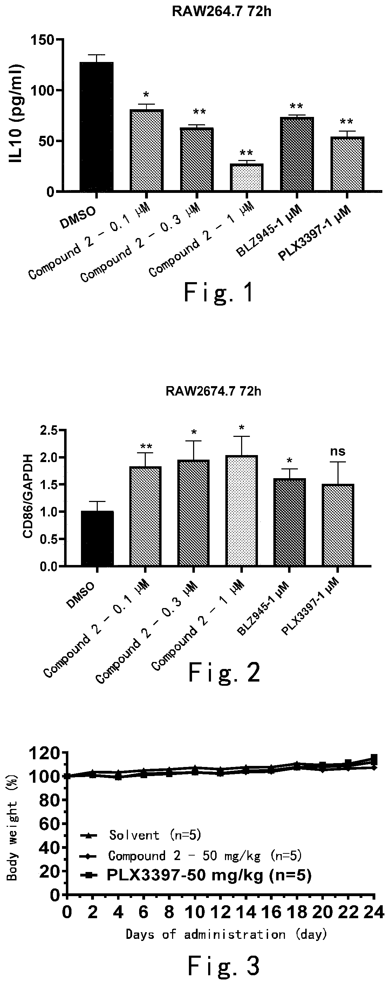

③ After the RT-PCR reaction reagents were prepared, they were mixed, and the mixture was dispensed into 8-Strip Tubes. 2 µL of RNA sample was added to the tube, the bottom of the tube was gently tapped to mix the solution, and the tube was centrifuged in a centrifuge for a while.
④ The fluorescence quantitative PCR instrument was turned on. After the instrument self-check was completed, the sample was put into the instrument and the PCR reaction was performed according to the following procedures: the first step was the reverse transcription reaction: 42°C 5 minutes, 95°C 10 seconds, 1 cycle; the second step was PCR amplification: 95°C 5 seconds, 60°C 20 seconds, 40 cycles.
⑤ After the experiment was completed, the sample was taken out and the instrument was turned off. Roche analysis software LightCycler^{®} 96 SW 1.1 was used to analyze the data, and the analyzed data was imported into Graphpad Prism7.0 for drawing. The results are shown in Figures 1 and 2.

The experiment showed that after the cells were induced to become M2 macrophages, the level of IL-10 secreted in M2 macrophages was much higher than that in M1 macrophages, which indicated that IL-10 was mainly secreted by M2 cells, while M1 macrophages secreted less. As shown in Figure 1, after addition of Compound 2 of the invention to treat M2 macrophages, the level of IL-10 secretion in M2 macrophages decreased in a dose-dependent manner as the drug concentration increased, indicating that among the macrophages M2 cells were gradually decreasing, and the effect was better for Compound 2 at 1 µM than positive controls PLX3397 and BLZ945 at the same dose. In addition, CD86 was a marker of M1 macrophages. As shown in Figure 2, after addition of Compound 2 to treat cells, it was found that the RNA level of CD86 increased in a dose-dependent manner as the drug concentration increased, indicating that the proportion of M1 cells in macrophages was gradually increasing. The effect was better for Compound 2 at 1 µM than positive controls PLX3397 and BLZ945 at the same dose. Since CSF1R kinase is a key protein that controls macrophage polarization in the tumor microenvironment, inhibiting the activity of CSF1R kinase can reduce the transformation of macrophages into M2 macrophages that promote cancer development, and increase the polarization of M1 macrophages that inhibit cancer development. This example proves that the compounds involved in the invention can reduce the transformation of macrophages into M2 macrophages and promote the transformation of macrophages into M1 macrophages, thereby inhibiting tumor growth and enhancing cancer immunotherapy.

### Example 43: In vivo efficacy assay

In this example, the experimental effects of Compound 2 and the control compound PLX3397 (purchased from MedChem Express, China) in MC38 and 4T1 mouse models were tested respectively.

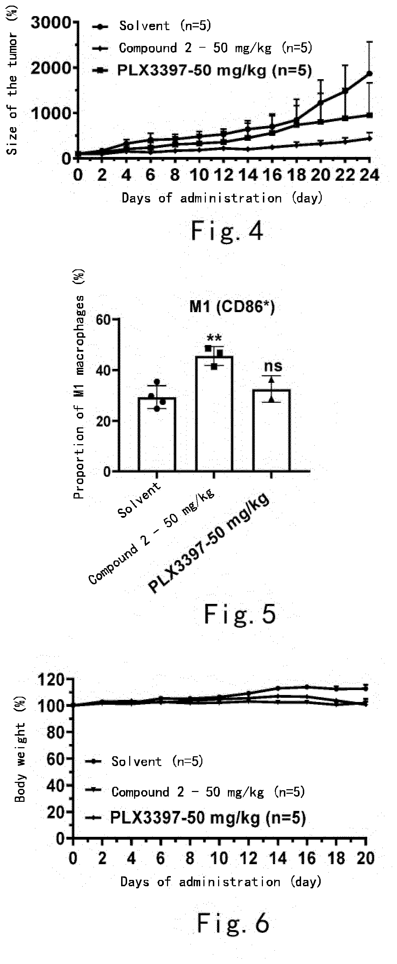

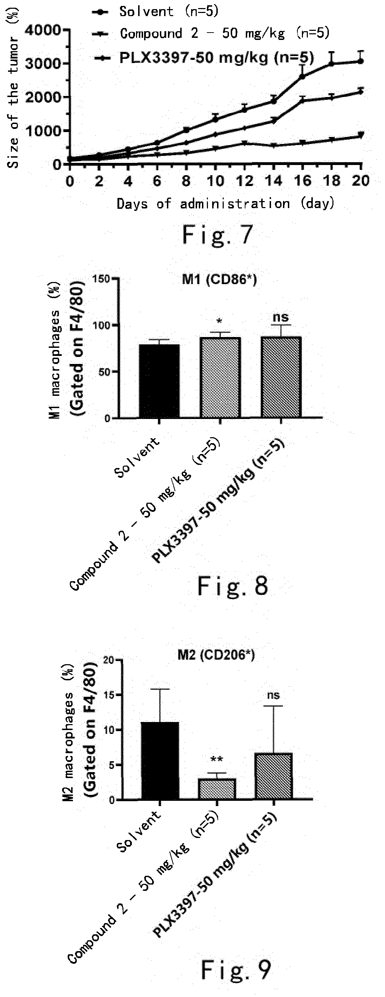

The experimental procedure was as follows:
(1) C57BL/6J male mice for use as MC38 transplanted tumor model and Balb/c male mice for use as 4T1 transplanted tumor model, which were 6 weeks old, were purchased from GemPharmatech Co., Ltd, Jiangsu. All the above mice were raised in an SPF laboratory, and the drinking water and padding had been sterilized by high pressure sterilization. All operations related to mice were performed under sterile conditions.
(2) On day 0, approximately 1×10⁶ MC38 cells (purchased from ATCC) were injected subcutaneously into the left back of each of the C57BL/6J mice, and approximately 1×10⁶ 4T1 cells (purchased from ATCC) were injected subcutaneously into the left back of each of the Balb/c mice.
(3) For the MC38 mouse model, starting from the 6th day, the corresponding mice were orally administered with methylcellulose (HKI) (purchased from China National Pharmaceutical Group Corporation) solvent once a day (5 mice); Compound 2 at a dose of 50 mg/kg mouse weight once a day (5 mice); PLX3397 at a dose of 50 mg/kg mouse weight once a day (5 mice). For the 4T1 mouse model, starting from the 6th day, the corresponding mice were orally administered with 10% HS-15 (purchased from BASF, Germany) solvent (5 mice); Compound 2 at a dose of 50 mg/kg mouse weight once a day (5 mice); PLX3397 at a dose of 50 mg/kg mouse weight once a day (5 mice).
(4) Starting from the 6th day, the length/width of the subcutaneous tumor was measured with a vernier caliper every day, and the weight of the mice was recorded every day to determine the effects of Compound 2 and control compound PLX3397 on the weight of mice in different model mice. The results are shown in Figures 3 and 6.
(5) The growth trends of subcutaneous tumors in mice of different models were statistically analyzed. The tumor volume was calculated using the following method: length × width × width/2 mm³. The results are shown in Figures 4 and 7.
(6) Isolation of tumor-infiltrating lymphocytes: Tumor tissue was first minced (tissue block less than 1 mm³) with a surgical blade, resuspended in 6 ml of digestion solution (collagenase IV and 0.005% DNasel enzyme, both purchased from Sigma, USA), and the mixture was transferred to a 15-ml centrifuge tube. The centrifuge tube was then placed in a shaker at 37 °C and digested with shaking at 220 r/min for 2 hours. The cells were sieved through a 200-mesh sieve and collected in a 50-ml centrifuge tube, and centrifuged at 1500 rpm for 10 minutes. The supernatant was then discarded, and 10 ml of DMEM medium (10% FBS) (purchased from GIBCO, USA) was added to the tube to terminate the digestion. The tube was placed in a centrifuge, and centrifuged at 1500 rpm for another 10 minutes. The supernatant was discarded. At the same time, Percoll separation solution (purchased from Millipore, USA) was prepared, and 90% Percoll separation working solution was prepared using 10×PBS. To a 15 ml centrifuge tube, 3-4 ml of 70% Percoll separation solution was added. The cells were resuspended in 4 ml of 40% Percoll separation solution, and the cell mixture was added to the 70% Percoll separation solution. The centrifuge tube was placed in a centrifuge and subjected to density gradient centrifugation at 25°C, 1260 g, for 30 minutes (speed up 5, speed down 0). After the centrifugation, the centrifuge tube was placed on a centrifuge tube rack, the middle leukocyte layer was aspirated with a pipette, and added to 10 ml of PBS, they were mixed well and placed in a centrifuge, and centrifuged at 1500 rpm. The cell supernatant was discarded after 10 min and the cells were resuspended in 5 ml of PBS, centrifuged at 1500 rpm for another 10 min, and the washing process was repeated once. The cells obtained were leukocytes in the tumor tissue. The cells were placed on ice for later use.
(7) Antibody Labeling for Flow Cytometry: The cells were labeled with 3 antibodies, first labeling markers on the cell surface, and then intracellular factors. Cell surface staining: 1×10⁶ cells were taken for centrifugation at 3500 rpm for 5 minutes, the cell supernatant was discarded. 1 ×PBS (50 µL) (purchased from Sangon Biotech Co., Ltd, China) was added to resuspend the cells. 1 µL of CD86 antibody and 1 µL of F4/80 antibody (CD86 and F4/80 antibodies were both purchased from BD Pharmingen, USA) were added into the tube, and the tube was incubated at 4°C for 30 minutes; then the tube was centrifuged at 3500 rpm for 5 minutes, the supernatant was discarded. The cells were washed twice with 1× staining buffer, and the cells were resuspended in 100 µL PBS for later use. Intracellular factor staining: the cells were treated with permeabilization/fixation solution. First, 250 µl of permeabilization/fixation solution (Cat. No. 554714, purchased from BD Biosciences, USA) was add to the cell suspension and the mixture was incubated on ice for 20 minutes; then, 1 ml of 1×BD Perm/Wash buffer was added to resuspend the cells and the resultant was placed into a centrifuge to centrifuge at 3500 rpm for 5 minutes; the cell supernatant was discarded; the procedures were repeated; then, 50 µl of 1×BD Perm/Wash buffer was added to the tube to resuspend the cells, and an appropriate amount of 1 µl CD206 antibody (both purchased from BD Pharmingen, USA) was added to the cell suspension, the mixture was incubated at 4°C for 30 minutes; the procedures were repeated; finally the cells were resuspended with PBS and detected on the machine. The data was processed using Flowjo, imported into Graphpad Prism software for plotting. The results are shown in Figures 5, 8, and 9.

The transplanted tumor models of C57BL/6J mouse MC38 and Balb/c mouse 4T1 cells are commonly used models of tumor immunity. After the drug activates the mouse's own tumor immunity following administration, the tumor growth is inhibited by the mouse's own immunity. The experimental results are shown in Figures 3-4 and 6-7. Compound 2 and the control compound PLX3397 had no obvious toxicity in the tumor immune models, MC38 and 4T1 mouse tumor models, and showed good inhibition effect of mouse tumors. As the days of administration increased, the inhibitory effect of Compound 2 on mouse tumors became more and more significant, which was superior to the effect of the control compound PLX3397.

In addition, as shown in Figures 5, 8 and 9, by detecting the proportion of macrophages in the tumor microenvironment, it was found that the proportion of M1 macrophages around the tumor in the group administered with Compound 2 was much higher than that in the solvent group, and at the same dose, the proportion of M1 macrophages in the tumor microenvironment of the group administered with Compound 2 was also higher than that of the PLX3397 group. This indicates that the compound of the invention can induce the transformation of M2 TAM into M1 TAM to inhibit tumor growth, and therefore can be used to treat cancers or tumors in which M2 macrophages are dominant in the tumor microenvironment.

### Industrial applicability

The invention provides a novel use of quinoline inhibitor compounds, which can polarize macrophages from M2 to M1 by inhibiting CSF1R kinase activity, thereby achieving the effect of treating cancer or tumors. Therefore, the invention is suitable for industrial applications.

Although the invention is described in detail herein, the invention is not limited thereto. Those skilled in the art can make modifications according to the principles of the invention. Therefore, any modifications made according to the principles of the invention should be understood to fall within protection scope of the invention.

## Claims

1. A compound of Formula (I) or a pharmaceutically acceptable salt, solvate, ester, acid, metabolite or prodrug thereof for use in treating cancers or tumors in which M2 macrophages are dominant in the tumor microenvironment: 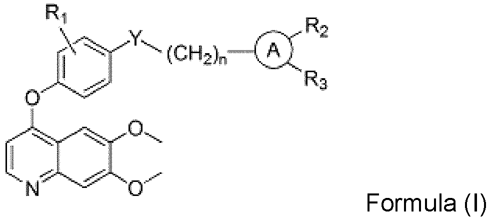
wherein, Y is selected from 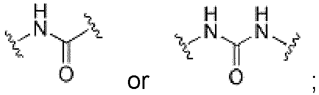 A is selected from aryl or 6-membered heterocyclyl;
n is an integer selected from 1-3;
R₁ is selected from hydrogen, halo, C₁₋₆alkyl, C₁₋₆haloalkyl, C₁₋₆alkoxy and cyano;
each of R₂ and R₃ is independently selected from hydrogen, halo, C₁₋₆alkyl, C₁₋₆haloalkyl, C₁₋₆alkoxy, or R₂ and R₃ together form a phenyl or 5-membered heterocyclyl.

2. The compound of Formula (I) or a pharmaceutically acceptable salt, solvate, ester, acid, metabolite or prodrug thereof for use according to claim 1, wherein A is selected from phenyl, N-morpholinyl, N-piperidyl or N-piperazinyl.

3. The compound of Formula (I) or a pharmaceutically acceptable salt, solvate, ester, acid, metabolite or prodrug thereof for use according to claim 1
or 2, wherein n is 1 when Y is 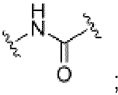 n is 3 when Y is

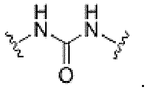

4. The compound of Formula (I) or a pharmaceutically acceptable salt, solvate, ester, acid, metabolite or prodrug thereof for use according to any one of claims 1-3, wherein R₁ is selected from hydrogen, fluoro, chloro, methyl, ethyl, propyl, or cyano; each of R₂ and R₃ is independently selected from hydrogen, fluoro, chloro, methyl, ethyl, propyl, trifluoromethyl, difluoromethyl, trifluoroethyl, methoxy, ethoxy, propoxy, or R₂ and R₃ together form a phenyl or dioxolane.

5. The compound of Formula (I) or a pharmaceutically acceptable salt, solvate, ester, acid, metabolite or prodrug thereof for use according to any one of claims 1-4, wherein the compound of Formula (I) is selected from a compound of Formula (Ia): 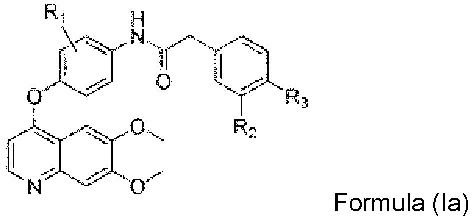 wherein R₁ is selected from hydrogen, fluoro, chloro, and methyl; each of R₂ and R₃ is independently selected from hydrogen, fluoro, chloro, methyl, and trifluoromethyl, or R₂ and R₃ together form a phenyl.

6. The compound of Formula (I) or a pharmaceutically acceptable salt, solvate, ester, acid, metabolite or prodrug thereof for use according to any one of claims 1-4, wherein the compound of Formula (I) is selected from a compound of Formula (Ib): 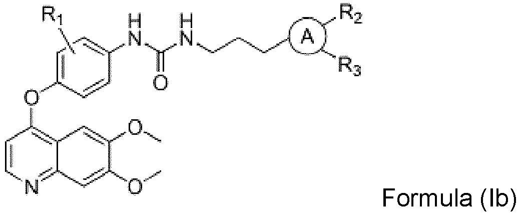 wherein A is selected from phenyl or N-morpholinyl; R₁ is hydrogen; each of R₂ and R₃ is independently selected from hydrogen, fluoro, chloro, methyl, and trifluoromethyl.

7. The compound of Formula (I) or a pharmaceutically acceptable salt, solvate, ester, acid, metabolite or prodrug thereof for use according to any one of claims 1-6, wherein the compound of Formula (I) is selected from the following compounds:
| | | | |
|---|---|---|---|
| 1 | 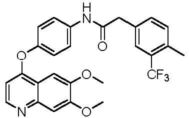 | 2 | 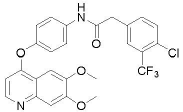 |
| 3 | 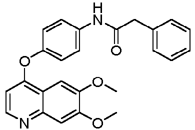 | 4 | 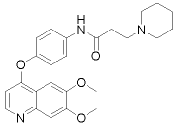 |
| 5 | 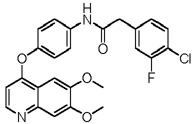 | 6 | 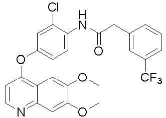 |
| 7 | 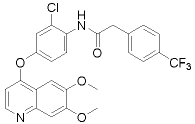 | 8 | 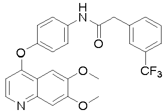 |
| 9 | 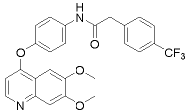 | 10 | 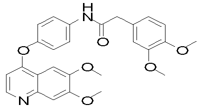 |
| 11 | 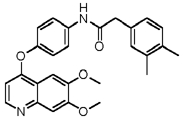 | 12 | 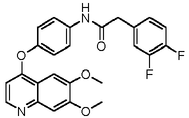 |
| 13 | 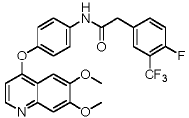 | 14 | 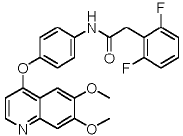 |
| 15 | 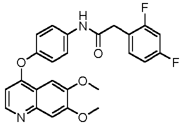 | 16 | 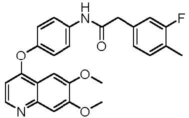 |
| 17 | 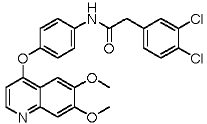 | 18 | 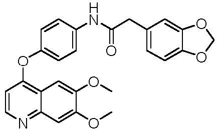 |
| 19 | 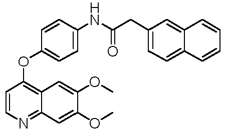 | 20 | 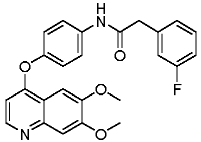 |
| 21 | 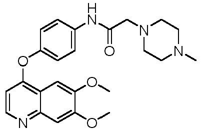 | 22 | 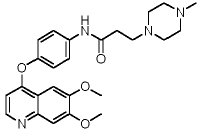 |
| 23 | 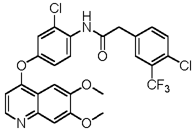 | 24 | 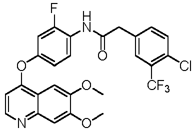 |
| 25 | 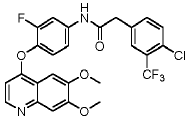 | 26 | 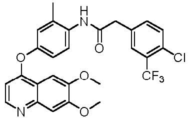 |
| 27 | 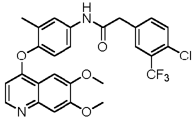 | 28 | 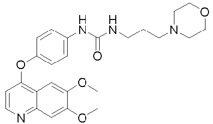 |
| 29 | 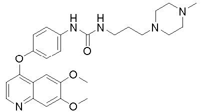 | 30 | 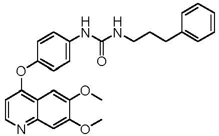 |
| 31 | 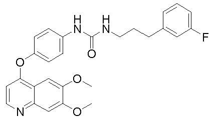 | 32 | 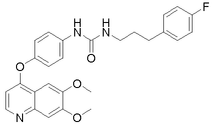 |
| 33 | 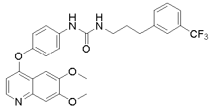 | 34 | 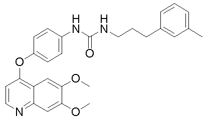 |
| 35 | 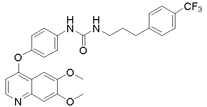 | 36 | 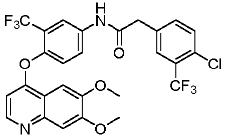 |
| 37 | 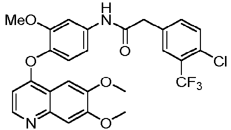 | 38 | 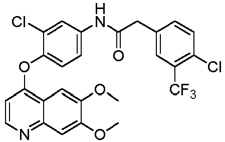 |
| 39 | 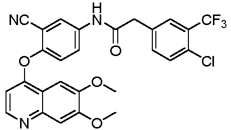 | 40 | 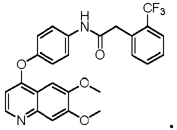 |

8. The compound of Formula (I) or a pharmaceutically acceptable salt, solvate, ester, acid, metabolite or prodrug thereof for use according to any one of claims 1-7, wherein,
the cancers or tumors are selected from one or more of ovarian cancer, cervical cancer, membranous adenocarcinoma, prostate cancer, bladder cancer, lung cancer, thyroid cancer, breast cancer, pancreatic cancer, kidney cancer, gastric cancer, liver cancer, cervix cancer, endometrial cancer, colorectal cancer, nasopharyngeal cancer, esophageal cancer, bile duct cancer, bone metastatic cancer, papillary thyroid cancer, non-small cell lung cancer, small cell lung cancer, colon cancer, solid tumor, brain tumor, lymphoma, glioma, glioblastoma, melanoma, mesothelioma, glioblastoma, osteosarcoma, gastrointestinal stromal tumor, multiple myeloma, carcinosarcoma of the bile duct, and leukemia.

9. The compound of Formula (I) or a pharmaceutically acceptable salt, solvate, ester, acid, metabolite or prodrug thereof for use according to claim 8, wherein the lymphoma is selected from non-Hodgkin's lymphoma, B cell or T cell lymphoma.

10. The compound of Formula (I) or a pharmaceutically acceptable salt, solvate, ester, acid, metabolite or prodrug thereof for use according to claim 8, wherein the leukemia is selected from chronic myelocytic leukemia or acute myeloid leukemia.

11. A method for treating cancers or tumors in which M2 macrophages are dominant in the tumor microenvironment, comprising administering to a patient a compound of Formula (I) or a pharmaceutically acceptable salt, solvate, ester, acid, metabolite or prodrug thereof in a therapeutically effective amount: 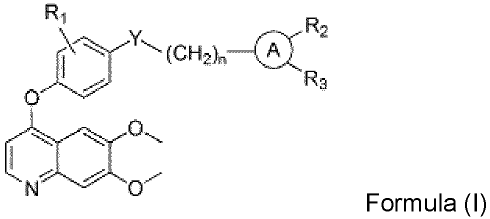
wherein, Y is selected from 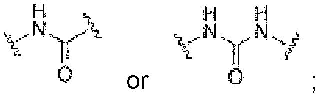
A is selected from aryl or 6-membered heterocyclyl;
n is an integer selected from 1-3;
R₁ is selected from hydrogen, halo, C₁₋₆alkyl, C₁₋₆haloalkyl, C₁₋₆alkoxy and cyano;
each of R₂ and R₃ is independently selected from hydrogen, halo, C₁₋₆alkyl, C₁₋₆haloalkyl, C₁₋₆alkoxy, or R₂ and R₃ together form a phenyl or 5-membered heterocyclyl.

12. The method according to claim 11, wherein A is selected from phenyl, N-morpholinyl, N-piperidyl or N-piperazinyl.

13. The method according to claim 11 or 12, wherein n is 1 when Y is 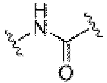 ; n is 3 when Y is

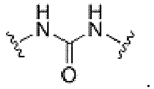

14. The method according to any one of claims 11-13, wherein R₁ is selected from hydrogen, fluoro, chloro, methyl, ethyl, propyl, or cyano; each of R₂ and R₃ is independently selected from hydrogen, fluoro, chloro, methyl, ethyl, propyl, trifluoromethyl, difluoromethyl, trifluoroethyl, methoxy, ethoxy, propoxy, or R₂ and R₃ together form a phenyl or dioxolane.

15. The method according to any one of claims 11-14, wherein the compound of Formula (I) is selected from a compound of Formula (Ia): 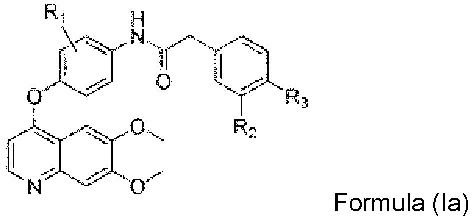 wherein R₁ is selected from hydrogen, fluoro, chloro, and methyl; each of R₂ and R₃ is independently selected from hydrogen, fluoro, chloro, methyl, and trifluoromethyl, or R₂ and R₃ together form a phenyl.

16. The method according to any one of claims 11-14, wherein the compound of Formula (I) is selected from a compound of Formula (Ib): 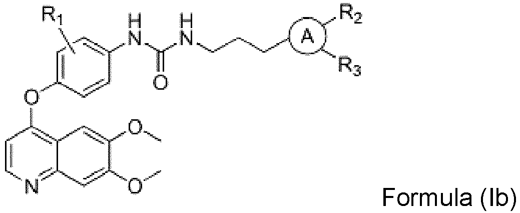 wherein A is selected from phenyl or N-morpholinyl; R₁ is hydrogen; each of R₂ and R₃ is independently selected from hydrogen, fluoro, chloro, methyl, and trifluoromethyl.

17. The method according to any one of claims 11-16, wherein the compound of Formula (I) is selected from the following compounds:
| | | | |
|---|---|---|---|
| 1 | 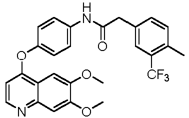 | 2 | 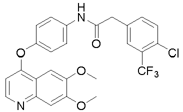 |
| 3 | 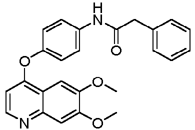 | 4 | 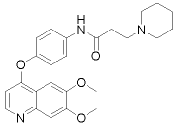 |
| 5 | 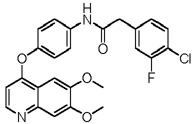 | 6 | 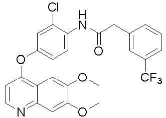 |
| 7 | 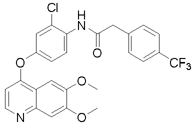 | 8 | 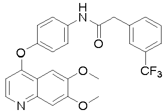 |
| 9 | 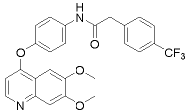 | 10 | 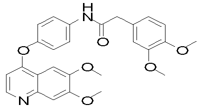 |
| 11 | 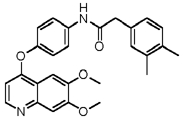 | 12 | 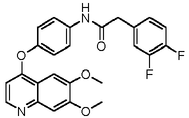 |
| 13 | 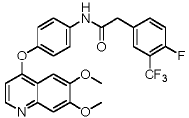 | 14 | 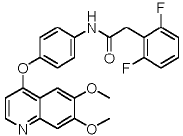 |
| 15 | 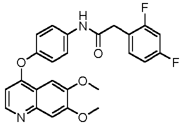 | 16 | 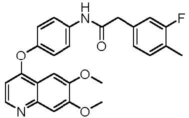 |
| 17 | 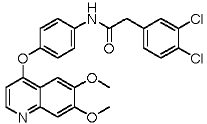 | 18 | 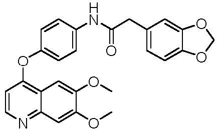 |
| 19 | 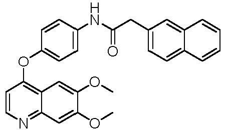 | 20 | 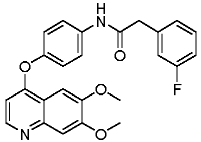 |
| 21 | 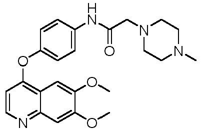 | 22 | 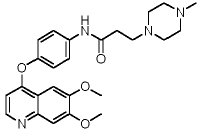 |
| 23 | 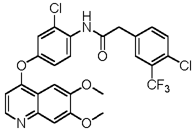 | 24 | 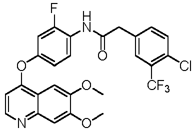 |
| 25 | 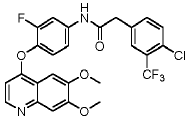 | 26 | 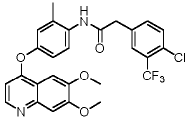 |
| 27 | 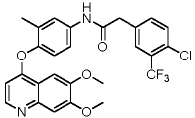 | 28 | 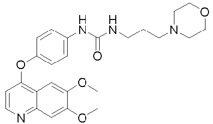 |
| 29 | 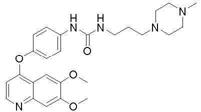 | 30 | 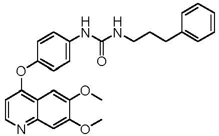 |
| 31 | 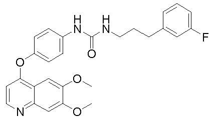 | 32 | 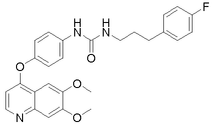 |
| 33 | 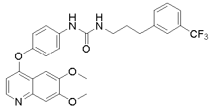 | 34 | 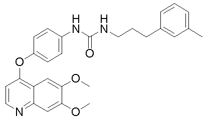 |
| 35 | 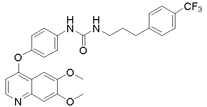 | 36 | 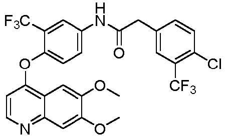 |
| 37 | 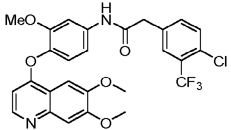 | 38 | 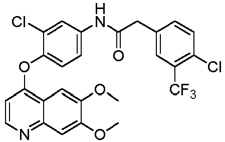 |
| 39 | 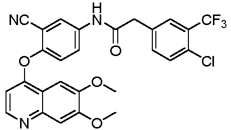 | 40 | 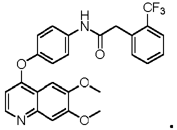 |

18. The method according to any one of claims 11-17, wherein,
the cancers or tumors are selected from one or more of ovarian cancer, cervical cancer, membranous adenocarcinoma, prostate cancer, bladder cancer, lung cancer, thyroid cancer, breast cancer, pancreatic cancer, kidney cancer, gastric cancer, liver cancer, cervix cancer, endometrial cancer, colorectal cancer, nasopharyngeal cancer, esophageal cancer, bile duct cancer, bone metastatic cancer, papillary thyroid cancer, non-small cell lung cancer, small cell lung cancer, colon cancer, solid tumor, brain tumor, lymphoma, glioma, glioblastoma, melanoma, mesothelioma, glioblastoma, osteosarcoma, gastrointestinal stromal tumor, multiple myeloma, carcinosarcoma of the bile duct, and leukemia.

19. The method according to claim 18, wherein the lymphoma is selected from non-Hodgkin's lymphoma, B cell or T cell lymphoma.

20. The method according to claim 18, wherein the leukemia is selected from chronic myelocytic leukemia or acute myeloid leukemia.
